# EUROPEAN PATENT SPECIFICATION

(11) **EP 1 509 511 B1**
(45) Date of publication and mention of the grant of the patent: **21.09.2005**
(21) Application number: 03752729.8
(22) Date of filing: 12.05.2003
(51) Int. Cl.: C07D 307/62, C07B 41/12, C07B 63/00, C07B 63/02, A61K 31/375

(54) **MANUFACTURE OF ASCORBIC ACID ESTERS**
HERSTELLUNG VON ASCORBINSÄUREESTERN
FABRICATION D'ESTERS D'ACIDE ASCORBIQUE

(30) Priority: 21.05.2002 EP 02011149
(43) Date of publication of application: 02.03.2005
(73) Proprietor: DSM IP Assets B.V., 6411 TE Heerlen (NL)
(72) Inventor: STAMM, Thomas, 79639 Grenzach-Wyhlen (DE)
(74) Representative: Schwander, Kuno
(86) International application number: PCT/EP2003/004907
(87) International publication number: WO 2003/097626

(56) References cited:
- COUSINS, RAYMOND C. ET AL: "Synthesis of 6-fatty acid esters of L-ascorbic acid" JOURNAL OF THE AMERICAN OIL CHEMISTS' SOCIETY (1977), 54(8), 308-12 , XP009013888
- UESATO, S. ET AL: "Inhibitory effects of 6-O-acylated l-ascorbic acids possessing a straight or branched-acyl chain on Epstein-Barr virus activation" CANCER LETTERS (SHANNON, IRELAND) (2001), 166(2), 143-146 , XP002247919
- YAN, YOUCHUN ET AL: "Lipase -catalyzed synthesis of vitamin C fatty acid esters" BIOTECHNOLOGY LETTERS (1999), 21(12), 1051-1054 , XP009013771

## Description

The present invention relates to a process for the manufacture of ascorbic acid esters of fatty acids, more particularly a process for the manufacture of ascorbic acid esters of saturated and unsaturated fatty acids, such as lauric, myristic, palmitic and stearic acids.

It is known that fatty acid esters of ascorbic acid can be manufactured by reacting ascorbic acid with a fatty acid, such as palmitic acid, or the methyl or ethyl ester thereof, in the presence of concentrated sulphuric acid, e.g. about 95% sulphuric acid or an oleum (highly concentrated sulphuric acid) such as one containing up to about 30% of added sulphur trioxide, pouring the reaction product onto ice or adding cold water to the reaction product, and recovering the desired ascorbic acid fatty acid ester (ascorbyl fatty acid ester) as a solid precipitate or by crystallization after extraction: see e.g. U.S. Patents 4,151,178 and 4,705,869.

As used throughout this specification the term "ascorbic acid" or "ascorbyl" is to be understood as referring to any isomer of ascorbic acid, such as the natural isomer, L-ascorbic acid, and D-isoascorbic acid, if not otherwise specified.

The reaction of ascorbic acid, or a salt thereof, with a fatty acid, or an ester or salt thereof, in the presence of concentrated sulphuric acid, besides yielding the desired ascorbic acid 6-fatty acid ester, e.g. L-ascorbyl-6-palmitate, produces by-products such as the ascorbic acid 5-fatty acid ester, e.g. L-ascorbyl-5-palmitate, and also the sulphates of the 6- and 5-esters, e.g. L-ascorbyl-6-palmitate or L-ascorbyl-5-palmitate 2- or 3-sulphate. In the usual work-up procedures, i.e. the isolation of the 6-ester by solid precipitation or extraction with a solvent such as diethyl ether followed by crystallization, the further esterified by-products such as the L-ascorbyl-5 or 6-palmitate 2-sulphate and/or 3-sulphate remain in the product and give rise to secondary formation of still further by-products. Hence, the pure 6-ester, e.g. L-ascorbyl-6-palmitate, is not obtained, and the starting ascorbic acid is not converted into the desired 6-fatty acid ester to an adequate extent.

It has now been found that in the sulphuric acid-catalysed esterification of ascorbic acid with fatty acids the ascorbic acid can be converted more efficiently into substantially pure ascorbyl-6-fatty acid ester when the esterification products are extracted with diethyl ketone, followed by hydrolysis of sulphate groups present in the ester sulphate by-products, removal of sulphuric acid from the product of this hydrolysis and, finally, isomerization of the ascorbic acid 5-fatty acid ester by-products into the desired ascorbic acid 6-fatty acid ester by acid catalysis in a non-polar aprotic organic solvent. In the foregoing statement and in any subsequent occurrence thereof the expression "substantially pure" in respect of the ascorbyl-6-fatty acid ester specifies a purity of at least 95%, particularly one of at least 98.5%

Accordingly, the present invention provides a process for the isolation of substantially pure ascorbyl-6-fatty acid ester from the products obtained by the sulphuric acid-catalyzed esterification of ascorbic acid with fatty acids which comprises extracting with diethyl ketone such reaction products from the mixture remaining after the esterification reaction, hydrolysing the sulphate ester by-products in the diethyl ketone extract, removing the generated sulphuric acid from the product of this hydrolysis, isomerizing the ascorbyl-5-fatty acid ester by-product to the desired ascorbyl-6-fatty acid ester by acid catalysis in a non-polar aprotic organic solvent, and recovering the accumulated ascorbyl-6-fatty acid ester.

As another aspect, the present invention provides a process for the manufacture of fatty acid esters of ascorbic acid, which process comprises the steps of
a) reacting ascorbic acid or an alkali metal or alkaline earth metal salt thereof with a fatty acid or with a lower allcyl ester or an alkali metal or alkaline earth metal salt thereof in concentrated sulphuric acid;
b) extracting the obtained esterification products from the mixture remaining after the reaction of step a), optionally after adding water to said mixture, with diethyl ketone;
c) hydrolysing any sulphate ester by-products in the diethyl ketone phase obtained in the extraction of step b);
d) removing sulphuric acid from the hydrolysed reaction product obtained in step c);
e) removing from the diethyl ketone phase present after step d) as much as possible of the diethyl ketone solvent;
f) adding a non-polar aprotic organic solvent and optionally some additional strong acid to the product of step e) and isomerizing the ascorbyl-5-fatty acid ester present to ascorbyl-6-fatty acid ester;
g) neutralizing the acid in the mixture present after the completion of step f); and
h) isolating the ascorbyl-6-fatty acid ester from the mixture present after the neutralization of step f).

The reaction of the ascorbic acid or its salt with the fatty acid or the ester or salt in the concentrated sulphuric acid [process step a)] can be performed in a known manner, e.g. as described in the above-mentioned references. Instead of ascorbic acid itself (free ascorbic acid), an alkali metal salt of ascorbic acid, such as the sodium or potassium salt, or an alkaline earth metal salt, such as the calcium salt, may be used. Preferably, however, free ascorbic acid is used. The fatty acid is suitably a saturated C₄₋₂₀-alkanoic acid, e.g. lauric, myristic, palmitic or stearic acid, preferably palmitic acid, but may also be an unsaturated C₄₋₂₀ fatty acid featuring one to three ethylenic double bonds, such as a polyunsaturated fatty acid (PUFA). Instead of the free fatty acid, an ester, suitably a lower alkyl ester, e.g. the methyl or ethyl ester, or an alkali metal or alkaline earth metal salt, preferably the sodium or calcium salt, respectively, may be used.

The sulphuric acid used may be 95% sulphuric acid or an oleum containing up to about 30 wt.% added sulphur trioxide, but is preferably sulphuric acid commonly designated as "95% sulphuric acid" or a higher concentrated variant up to "100.5 wt.% sulphuric acid".

Suitably, the fatty acid or its lower alkyl ester or alkali metal or alkaline earth metal salt is reacted in excess, e.g. in an about 20% to about 100% molar excess relative to the molar amount of ascorbic acid or its salt which is used. Preferably, an about 25% molar excess is used. Moreover, the esterification is suitably carried out at room temperature or slightly elevated temperature, i.e. generally in the range from about 20°C to about 30°C. Depending on the reaction temperature, the esterification reaction of step a) is usually complete within about 8 to 12 hours.

In the next process step [b)] the mixture remaining after the esterification reaction is extracted with diethyl ketone. Since said mixture is generally very viscous, it is suitably diluted with water, preferably water cooled to as low as 0°C to avoid undesired further reactions, and suitably with an about threefold to about sixfold amount by weight thereof, prior to the extraction with diethyl ketone. In adding the cold water, the temperature of the mixture being diluted is suitably maintained in the range of about 0°C to about 5°C. The diluted, less viscous mixture is then extracted with diethyl ketone, suitably with an about threefold to about fivefold amount by volume of said ketone. The diethyl ketone phase is finally separated from the aqueous phase, which can be carried out conventionally.

In the following process step [c)] the separated diethyl ketone phase is suitably maintained at elevated temperature, e.g. a temperature of about 30°C to about 80°C, preferably about 55°C to about 70°C, for a period of time sufficient to hydrolyse off sulphate ester groups from the sulphated ascorbyl-5- and/or 6-fatty acid ester contained in the product of steps a) and b). The progress of the hydrolysis in the heated diethyl ketone extract can be monitored by conventional analytical means, e.g. by high pressure liquid chromatography (HPLC) or by thin layer chromatography (TLC). Typically, at 60°C, the hydrolysis is completed within about 30 minutes.

Subsequently, in the process step d), the diethyl ketone phase is substantially freed from the sulphuric acid present therein at least partly as a result of its generation in the previous heat treatment step. The removal of this sulphuric acid is conveniently effected by washing the diethyl ketone with water and/or by treating it with a solid weak, substantially insoluble base, such as solid calcium or magnesium carbonate or a solid weakly basic ion exchange resin, e.g. XE 654 (Rohm and Haas). Where washing with water followed by treatment with a solid base is employed, the aqueous phase from the washing must be separated off before the base is added. After treatment with a solid base the solid components remain in the organic phase, and these must be removed, suitably by filtration. If, as an alternative, passage of the diethyl ketone extract over the base is effected, particularly where a solid weakly basic ion exchange resin is employed, the presence of solid components may be substantially avoided and, accordingly, subsequent filtration is rendered unnecessary. If desired, treatment with a solid base may be followed by washing with water, in which case the diethyl ketone phase is finally separated from the aqueous phase; this can be carried out conventionally. The isolated diethyl ketone phase, freed of solid components and/or separated from the aqueous phase, generally imparts a pH of about 3.0 to 4.5 to a third of its volume of a water extract used conveniently to monitor the extent of acid removal, and contains the desired ascorbyl-6-fatty acid ester as well as some ascorbyl-5-fatty acid ester, which is later [(in process step f)] isomerized to the 6-fatty acid ester.

In the next step [e)], the diethyl ketone solvent is then removed, suitably in conventional manner by evaporation under reduced pressure and at elevated temperature. In this way 98 to 100% of the solvent can be removed as a rule.

The isomerization effected in the next stage, step f), can be effected by the action of traces of a strong acid, remaining from the previous steps d) and e), in the presence of a non-polar aprotic organic solvent. Suitably, a non-polar aprotic organic solvent is,added to the mixture resulting from the removal of diethyl ketone solvent effected in step e). The solvent medium for the isomerization process should be essentially non-polar since this favours the formation of the ascorbyl-6-fatty acid ester which in non-polar solvents is generally less soluble than the 5-ester; moreover, an essentially non-polar solvent system largely suppresses any isomerization of the 6-fatty acid ester to the 5-fatty acid ester which might otherwise occur. Suitable non-polar aprotic organic solvents for use in this step f) are lower alkanes, particularly hexane, and aromatic hydrocarbons, e.g. benzene and toluene. Preferably, the solvent system comprises at least about 9% by volume of the added solvent, said solvent preferably being hexane, the rest being remaining diethyl ketone. While traces of sulphuric acid still present in the diethyl ketone extract may well suffice as the acid to catalyze the isomerization, it may be required to supplement the amount of acid by adding a small amount of a strong acid such as a mineral acid, e.g. (additional) sulphuric acid, hydrochloric acid, a hydrogen sulphate, e.g. sodium hydrogen sulphate, or a strongly acidic ion exchange resin. The mixture is suitably then maintained at room temperature or at slightly elevated temperature, e.g. at a temperature up to about 60°C, until the isomerization has been completed. The time required for the isomerization depends inter alia on the temperature of the reaction mixture and may vary from about 6 hours at room temperature to about 3 hours at 60°C. In any event the progress of the isomerization of the ascorbyl-5-fatty acid ester to the desired ascorbyl-6-fatty acid ester can be monitored by conventional analytical techniques, e.g. HPLC or TLC.

When the isomerization of the ascorbyl-5-fatty acid ester to the desired ascorbyl-6-fatty acid ester has been completed the reaction mixture is neutralized in the following step, g), e.g. by the addition of an alkaline earth metal carbonate, preferably calcium carbonate. The agent added for neutralization, e.g. calcium carbonate, is conveniently added in suspension in a non-polar organic solvent, e.g. hexane. There is obtained a suspension containing the desired ascorbyl-6-fatty acid ester and the appropriate alkaline earth metal sulphate.

To subsequently isolate the ascorbyl-6-fatty acid ester in the final step, h), diethyl ketone is suitably added to the neutralized suspension obtained in step g) in an amount to dissolve the ascorbyl-6-fatty acid ester. Charcoal may also suitably be added for purification purposes. The pure ascorbyl-6-fatty acid ester is obtained suitably by filtration of the warm mixture and cooling the filtrate, the resulting precipitate, generally in crystalline form, of ascorbyl-6-fatty acid ester then being isolated, conveniently by filtration. If desired, and in the case where a higher fatty acid, e.g. palmitic acid, is used as the fatty acid, the mother liquor can then be concentrated to recover the excess of the appropriate fatty acid present as a by-product, e.g. palmitic acid, as a precipitate, which can then be recycled if desired.

To achieve an optimal yield at all stages of the process of the present invention all operations should be carried out in an inert atmosphere, e.g. under nitrogen, and the solvents used should be gassed with inert gas, e.g. nitrogen, before use. Furthermore, quick processing of reaction steps b), c) and d) is preferred to this end.

### The following Example illustrates the invention:

### Example

### A. Esterification [process step a)]

1.02 kg of ascorbic acid and 1.85 kg of palmitic acid were added to 4.7 1 of oleum (100.5% of sulphur trioxide) with stirring and cooling. The reaction was allowed to proceed for 12 hours at 30 °C (jacket temperature).

### B. Dilution and extraction [process step b)]

In this and the following steps all the solvents were gassed with nitrogen before use and all the operations were carried out under nitrogen. The viscous mixture obtained after completion of the previous reaction step (esterification) and the threefold amount of deionized ice water were introduced simultaneously with vigorous stirring into a vessel while maintaining the temperature between 0°C and 2°C. The resulting diluted, less viscous mixture was then extracted with 1001 of diethyl ketone at a temperature not exceeding 20-25°C.

### C. Hydrolysis of sulphate esters [process step c)]

The separated diethyl ketone extract of the previous step was heated to 65°C until the amount of product containing sulphate ester groups was less than 0.5%, as monitored by HPLC/TLC.

### D. Removal of sulphuric acid [process step d)]

The separated heat-treated diethyl ketone extract resulting from the previous step was cooled to 20-22°C and washed with deionized water. Then, after removal of the aqueous phase, the separated diethyl ketone phase was passed through a bed of the weakly basic ion exchange resin XE 654 (Rohm and Haas) until the pH of an aqueous extract (obtained with 50 ml of water from a 150 ml aliquot of the diethyl ketone phase) was about 3.7- 3.8.

### E. Evaporation of solvent [process step e)]

The diethyl ketone phase was evaporated at 60 mbar (6 kPa) and 45°C to a solid residue, and this was dried at 10 mbar (1 kPa) and 60°C (jacket temperature).

### F. Isomerization [process step f)]

301 of hexane and 5 g of concentrated sulphuric acid were added to the residue obtained after completion of the previous step, and the suspension was heated at 60°C for 3 hours.

### G. Neutralization [process step g)]

A suspension of 5.2 g of calcium carbonate in 200 ml of hexane was added to the mixture resulting from the previous step, and the augmented mixture was stirred for 30 minutes.

### H. Isolation [process step h)]

121 of diethyl ketone at 60°C were added to the mixture resulting in the previous step, followed by a slurry of 144 g of charcoal in 11 of diethyl ketone to the resulting solution at 60°C. After filtration of the hot solution and washing of the filter cake with 5 1 of diethyl ketone 61 of hexane were added. The solution was then cooled stepwise with stirring to 15°C, then to 3-4°C. The resulting precipitate was separated by centrifugation, washed with 201 of an equivolume mixture of diethyl ketone and hexane at 3-4°C and dried at 50°C under reduced pressure. The crystalline product consisted of substantially pure ascorbyl-6-palmitate. The mother liquor was evaporated to dryness at 60°C/100 mbar (10 kPa) and the residue was recrystallized from diethyl ketone.

## Claims

1. A process for the isolation of a pure ascorbyl-6-fatty acid ester from the products obtained by the sulphuric acid-catalyzed esterification of ascorbic acid with a fatty acid, comprising extracting with diethyl ketone such reaction products from the mixture remaining after the reaction, hydrolysing the sulphate ester by-products, being sulphated ascorbyl-5- and/or 6-fatty acid ester, in the diethyl ketone extract by maintaining the extract at a temperature of about 30°C to about 80°C for a period of time sufficient to hydrolyse off sulphate ester groups from said sulphate ester by-products, removing the generated sulphuric acid from the product of this hydrolysis by washing the diethyl ketone phase with water and/or by treating it with a solid weak, substantially insoluble base, isomerizing the ascorbyl-5-fatty acid ester by-products to the desired ascorbyl-6-fatty acid ester by acid catalysis in a non-polar aprotic organic solvent, and recovering the accumulated ascorbyl-6-fatty acid ester.

2. A process for the manufacture of fatty acid esters of ascorbic acid, which process comprises the steps of
a) reacting ascorbic acid or an alkali metal or alkaline earth metal salt thereof with a fatty acid or with a lower alkyl ester or an alkali metal or alkaline earth metal salt thereof in concentrated sulphuric acid;
b) extracting the obtained esterification products from the mixture remaining after the reaction of step a), optionally after adding water to said mixture, with diethyl ketone;
c) hydrolysing any sulphate ester by-products, being sulphated ascorbyl-5- and/or 6-fatty acid ester, in the diethyl ketone phase obtained in the extraction of step b) by maintaining the extract at a temperature of about 30°C to about 80°C for a period of time sufficient to hydrolyse off sulphate ester groups from said sulphate ester by-products;
d) removing sulphuric acid from the hydrolysed reaction product obtained in step c) by washing the diethyl ketone phase with water and/or by treating it with a solid weak, substantially insoluble base;
e) removing from the diethyl ketone phase present after step d) as much as possible of the diethyl ketone solvent;
f) adding a non-polar aprotic organic solvent and optionally some additional strong acid to the product of step e) and isomerizing the ascorbyl-5-fatty acid ester present to ascorbyl-6-fatty acid ester;
g) neutralizing the acid in the mixture present after the completion of step f); and
h) isolating the ascorbyl-6-fatty acid ester from the mixture present after the neutralization of step f).

3. A process according to claim 2, wherein in step a) there is used as the alkali metal or alkaline earth metal salt of the ascorbic acid the sodium or potassium salt, or, respectively, the calcium salt, and as the lower alkyl ester or alkali metal salt of the fatty acid the methyl or ethyl ester, or, respectively, the sodium salt.

4. A process according to any one of claims 1 to 3, wherein the fatty acid is a saturated C₄₋₂₀-alkanoic acid, preferably palmitic acid, or an unsaturated C₄₋₂₀-fatty acid featuring one to three ethylenic double bonds, preferably a polyunsaturated fatty acid.

5. A process according to any one of claims 2 to 4, wherein in step a) the fatty acid or its lower alkyl ester or alkali metal or alkaline earth metal salt is reacted in excess, preferably in an about 20% to about 100% molar excess, more preferably in an about 25% molar excess, relative to the molar amount of ascorbic acid or its salt which is used.

6. A process according to any one of claims 2 to 5, wherein in step a) the sulphuric acid used is 95% sulphuric acid or a higher concentrated variant up to 100.5% sulphuric acid.

7. A process according to any one of claims 1 to 6, wherein before the extraction with diethyl ketone [step b)] an about threefold to about sixfold amount by weight of water is added to the mixture prior to the extraction with diethyl ketone.

8. A process according to any one of claims 1 to 7, wherein before the extraction with diethyl ketone [step b)] cold water is added while keeping the temperature of the mixture being diluted in the range of about 0°C to about 5°C.

9. A process according to any one of claims 1 to 8, wherein before the extraction with diethyl ketone [step b)] cold water is added and the diluted, less viscous mixture is then extracted with an about threefold to about fivefold amount by volume of the diethyl ketone.

10. A process according to any one of claims 1 to 9, wherein in the hydrolysis of the sulphate ester by-products in the diethyl ketone extract [step c)] the separated diethyl ketone extract is maintained at a temperature of about 55°C to about 70°C.

11. A process according to any one of claims 1 to 10, wherein after the washing with water and/or treatment with a solid weak, substantially insoluble base of the diethyl ketone phase [step d)] any aqueous phase from the washing and/or any solid components remaining from the treatment with base are removed.

12. A process according to claim 11, wherein the base is solid calcium or magnesium carbonate or XE 654 (Rohm and Haas).

13. A process according to any one of claims 2 to 12, wherein in step e) the diethyl ketone solvent is removed by evaporation under reduced pressure and at elevated temperature.

14. A process according to any one of claims 1 to 13, wherein in the isomerization of the ascorbyl-5-fatty acid ester by-products to the desired ascorbyl-6-fatty acid ester by acid catalysis in a non-polar aprotic organic solvent [step f)] the non-polar aprotic organic solvent is a lower alkane, preferably hexane, or an aromatic hydrocarbon, preferably benzene or toluene.

15. A process according to any one of claims 1 to 14, wherein for the isomerization of the ascorbyl-5-fatty acid ester by-products to the desired ascorbyl-6-fatty acid ester by acid catalysis in a non-polar aprotic organic solvent [step f)] additional strong acid is added and this is a mineral acid, preferably (additional) sulphuric acid, hydrochloric acid or a hydrogen sulphate, or a strongly acidic ion exchange resin.

16. A process according to any one of claims 2 to 15, wherein in step g) the neutralization is effected by the addition of an alkaline earth metal carbonate, preferably calcium carbonate.

17. A process according to any one of claims 2 to 16, wherein in step h) the isolation of the ascorbyl-6-fatty acid ester from the mixture present after the neutralization is effected by adding diethyl ketone to the neutralized suspension in an amount to dissolve the ascorbyl-6-fatty acid ester, optionally also adding charcoal, filtering the warmed mixture, cooling the filtrate and isolating the resulting precipitate of ascorbyl-6-fatty acid ester, preferably by filtration.

18. A process according to any one of claims 2 to 17, wherein L-ascorbyl palmitate is manufactured and isolated by precipitation, and the by-product palmitic acid is recovered from the mother liquor of the L-ascorbyl palmitate precipitation by concentrating said mother liquor.

## Patentansprüche

1. Verfahren zur Isolierung eines reinen Ascorbyl-6-fettsäureesters aus den Produkten, die durch die Schwefelsäure-katalysierte Veresterung von Ascorbinsäure mit Fettsäure erhalten wurden, umfassend die Extraktion mit Diethylketon solcher Reaktionsprodukte aus dem Gemisch, das nach der Reaktion verbleibt, die Hydrolyse der Sulfatesternebenprodukte, die sulfatierte Ascorbyl-5- und/oder 6-Fettsäureester sind, in dem Diethylketonextrakt, indem der Extrakt bei einer Temperatur von etwa 30 bis etwa 80 °C für einen Zeitraum gehalten wird, der ausreicht, die Sulfatestergruppen aus den Sulfatestemebenprodukten abzuhydrolysieren, die Entfernung der erzeugten Schwefelsäure aus dem Produkt dieser Hydrolyse durch Waschen der Diethylketonphase mit Wasser und/oder durch Behandlung mit einer festen schwachen, im wesentlichen unlöslichen Base, die Isomerisierung der Ascorbyl-5-fettsäureester-Nebenprodukte zu dem gewünschten Ascorbyl-6-fettsäureester durch Säurekatalyse in einem nicht polaren aprotischen organischen Lösungsmittel und die Gewinnung des angesammelten Ascorbyl-6-fettsäureesters.

2. Verfahren zur Herstellung von Fettsäureestern von Ascorbinsäure, wobei das Verfahren die Schritte:
a) Umsetzung von Ascorbinsäure oder einem Alkalimetall- oder Erdalkalimetallsalz hiervon mit einer Fettsäure oder mit einem Niederalkylester oder einem Alkalimetall- oder Erdalkalimetallsalz hiervon in konzentrierter Schwefelsäure;
b) Extraktion der erhaltenen Veresterungsprodukte aus dem Gemisch, das nach der Umsetzung von Schritt a) verbleibt, gegebenenfalls nach der Zugabe von Wasser zu dem Gemisch, mit Diethylketon;
c) Hydrolyse jeglicher Sulfatestemebenprodukte, die sulfatierte Ascorbyl-5- und/oder 6-Fettsäureester sind, in der Diethylketonphase, die in dem Extraktionsschritt b) erhalten wurde, indem der Extrakt bei einer Temperatur von etwa 30 bis etwa 80 °C für einen Zeitraum gehalten wird, der ausreicht, die Sulfatestergruppen aus den Sulfatesternebenprodukten abzuhydrolysieren;
d) Entfernung der Schwefelsäure aus dem hydrolysierten Reaktionsprodukt, das in Schritt c) erhalten wurde, durch Waschen der Diethylketonphase mit Wasser und/oder Behandlung mit einer festen, schwachen, im wesentlichen unlöslichen Base;
e) Entfernung von so viel wie möglich an Diethylketonlösungsmittel aus der Diethylketonphase, die nach Schritt d) vorhanden ist;
f) Zugabe eines nicht polaren aprotischen organischen Lösungsmittels und gegebenenfalls etwas zusätzlicher starker Säure zu dem Produkt aus Schritt e) und Isomerisierung des vorhandenen Ascorbyl-5-fettsäureesters zu Ascorbyl-6-fettsäureester;
g) Neutralisierung der Säure in dem Gemisch, das nach der Beendigung von Schritt f) vorhanden ist, und
h) Isolierung des Ascorbyl-6-fettsäureesters aus dem Gemisch, das nach der Neutralisierung von Schritt f) vorhanden ist, umfaßt.

3. Verfahren nach Anspruch 2, wobei in Schritt a) als das Alkalimetall- oder Erdalkalimetallsalz von Ascorbinsäure das Natrium- oder Kaliumsalz bzw. das Calciumsalz und als der Niederalkylester oder das Alkalimetallsalz der Fettsäure der Methyl- oder Ethylester bzw. das Natriumsalz verwendet wird.

4. Verfahren nach einem der Ansprüche 1 bis 3, wobei die Fettsäure eine gesättigte C₄₋₂₀-Alkansäure, bevorzugt Palmitinsäure, oder eine ungesättigte C₄₋₂₀-Fettsäure mit ein bis drei ethylenischen Doppelbindungen, bevorzugt eine mehrfach ungesättigte Fettsäure ist.

5. Verfahren nach einem der Ansprüche 2 bis 4, wobei in Schritt a) die Fettsäure oder ihr Niederalkylester oder Alkalimetall- oder Erdalkalimetallsalz in Überschuß, bevorzugt in einem etwa 20%igen bis etwa 100%igen molaren Überschuß, stärker bevorzugt in einem etwa 25%igen molaren Überschuß, bezogen auf die verwendete molare Menge an Ascorbinsäure oder ihres Salzes, umgesetzt wird.

6. Verfahren nach einem der Ansprüche 2 bis 5, wobei in Schritt a) die verwendete Schwefelsäure 95%ige Schwefelsäure oder eine höher konzentrierte Variante mit bis zu 100,5 % Schwefelsäure ist.

7. Verfahren nach einem der Ansprüche 1 bis 6, wobei vor der Extraktion mit Diethylketon [Schritt b)] eine etwa drei- bis etwa sechsfache Menge Wasser, bezogen auf das Gewicht, zu dem Gemisch vor der Extraktion mit Diethylketon zugegeben wird.

8. Verfahren nach einem der Ansprüche 1 bis 7, wobei vor der Extraktion mit Diethylketon [Schritt b)] kaltes Wasser zugegeben wird, während die Temperatur des Gemisches, das verdünnt wird, im Bereich von etwa 0 bis etwa 5 °C gehalten wird.

9. Verfahren nach einem der Ansprüche 1 bis 8, wobei vor der Extraktion mit Diethylketon [Schritt b)] kaltes Wasser zugegeben wird, und das verdünnte, weniger viskose Gemisch dann mit einer etwa dreifachen bis etwa fünffachen Menge Diethylketon, bezogen auf das Volumen, extrahiert wird.

10. Verfahren nach einem der Ansprüche 1 bis 9, wobei bei der Hydrolyse der SulfatesterNebenprodukte in dem Diethylketonextrakt [Schritt c)] der abgetrennte Diethylketonextrakt bei einer Temperatur von etwa 55 °C bis etwa 70 °C gehalten wird.

11. Verfahren nach einem der Ansprüche 1 bis 10, wobei nach der Wäsche der Diethylketonphase [Schritt d)] mit Wasser und/oder der Behandlung mit einer festen, schwachen, im wesentlichen unlöslichen Base jegliche wässerige Phase aus der Wäsche und/oder jegliche festen Komponenten, die aus der Behandlung mit der Base zurückbleiben, entfernt werden.

12. Verfahren nach Anspruch 11, wobei die Base festes Calcium- oder Magnesiumcarbonat oder XE 654 (Rohm and Haas) ist.

13. Verfahren nach einem der Ansprüche 2 bis 12, wobei in Schritt e) das Diethylketonlösungsmittel durch die Verdampfung unter vermindertem Druck und bei erhöhter Temperatur entfernt wird.

14. Verfahren nach einem der Ansprüche 1 bis 13, wobei bei der Isomerisierung der Ascorbyl-5-fettsäureester-Nebenprodukte zu dem gewünschten Ascorbyl-6-fettsäureester durch Säurekatalyse in einem nicht polaren aprotischen organischen Lösungsmittel [Schritt f)] das nicht polare aprotische organische Lösungsmittel ein Niederalkan, bevorzugt Hexan, oder ein aromatischer Kohlenwasserstoff, bevorzugt Benzol oder Toluol, ist.

15. Verfahren nach einem der Ansprüche 1 bis 14, wobei für die Isomerisierung der Ascorbyl-5-fettsäureester-Nebenprodukte zu dem gewünschten Ascorbyl-6-fettsäureester durch Säurekatalyse in einem nicht polaren aprotischen organischen Lösungsmittel [Schritt f)] zusätzliche starke Säure zugegeben wird, und diese eine Mineralsäure, bevorzugt (zusätzliche Schwefelsäure), Salzsäure, oder Hydrogensulfat oder ein stark saures Ionenaustauschharz ist.

16. Verfahren nach einem der Ansprüche 2 bis 15, wobei in Schritt g) die Neutralisation durch die Zugabe eines Erdalkalimetallcarbonats, bevorzugt Calciumcarbonat, herbeigeführt wird.

17. Verfahren nach einem der Ansprüche 2 bis 16, wobei in Schritt h) die Isolierung des Ascorbyl-6-fettsäureesters aus dem Gemisch, das nach der Neutralisation vorhanden ist, durch die Zugabe von Diethylketon zu der neutralisierten Suspension in einer Menge, mit der der Ascorbyl-6-fettsäureester aufgelöst wird, gegebenenfalls auch durch Zugabe von Holzkohle, Filtration des erwärmten Gemisches, Abkühlen des Filtrats und Isolierung des resultierenden Niederschlags des Ascorbyl-6-fettsäureesters, bevorzugt durch Filtration, herbeigeführt wird.

18. Verfahren nach einem der Ansprüche 2 bis 17, wobei L-Ascorbylpalmitat hergestellt und durch Ausfällung isoliert wird, und das Nebenprodukt Palmitinsäure aus der Stammlösung des L-Ascorbylpalmitatniederschlags durch Konzentrieren der Stammlösung gewonnen wird.

## Revendications

1. Procédé visant à isoler un ester d'acide gras d'ascorbyle-6 pur à partir des produits obtenus par l'estérification de l'acide ascorbique avec un acide gras catalysée par l'acide sulfurique, comprenant l'extraction de ces produits de réaction avec de la diéthylcétone à partir du mélange obtenu après la réaction, l'hydrolyse des sous-produits d'ester sulfate, qui sont l'ester d'acide gras d'ascorbyle-5 et/ou d'ascorbyle-6 sulfaté, dans l'extrait de diéthylcétone en maintenant l'extrait à une température d'environ 30°C à environ 80°C pendant une période de temps suffisante pour hydrolyser les groupes esters sulfates desdits sous produits d'ester sulfate, l'élimination de l'acide sulfurique généré à partir du produit de cette hydrolyse en lavant la phase de diéthylcétone avec de l'eau et/ou en la traitant avec une base faible solide sensiblement insoluble, l'isomérisation des sous-produits d'ester d'acide gras d'ascorbyle-5 en ester d'acide gras d'ascorbyle-6 désiré par catalyse acide dans un solvant organique aprotique non polaire, et la récupération de l'ester d'acide gras d'ascorbyle-6 accumulé.

2. Procédé de fabrication d'esters d'acide gras de l'acide ascorbique, lequel procédé comprend les étapes consistant à
a) faire réagir de l'acide ascorbique ou un sel de métal alcalin ou de métal alcalino-terreux de celui-ci avec un acide gras ou avec un ester alkylique inférieur ou un sel de métal alcalin ou de métal alcalino-terreux de celui-ci dans de l'acide sulfurique concentré ;
b) à extraire les produits d'estérification obtenus à partir du mélange obtenu après la réaction de l'étape a), éventuellement après avoir ajouté de l'eau audit mélange, avec de la diéthylcétone ;
c) à hydrolyser tous les sous-produits d'ester sulfate, qui sont l'ester d'acide gras d'ascorbyle-5 et/ou d'ascorbyle-6 sulfaté, dans la phase de diéthylcétone obtenue lors de l'extraction de l'étape b) en maintenant l'extrait à une température d'environ 30°C à environ 80°C pendant une période de temps suffisante pour hydrolyser les groupes esters sulfates desdits sous produits d'ester sulfate ;
d) à éliminer l'acide sulfurique du produit de réaction hydrolysé obtenu à l'étape c) en lavant la phase de diéthylcétone avec de l'eau et/ou en la traitant avec une base faible solide sensiblement insoluble ;
e) à éliminer de la phase de diéthylcétone présente après l'étape d) la plus grande partie du solvant diéthylcétone ;
f) à ajouter un solvant organique aprotique non polaire et éventuellement de l'acide fort supplémentaire au produit de l'étape e) et à isomériser l'ester d'acide gras d'ascorbyle-5 présent en ester d'acide gras d'ascorbyle-6 ;
g) à neutraliser l'acide dans le mélange présent après avoir accompli l'étape f) ; et
h) à isoler l'ester d'acide gras d'ascorbyle-6 à partir du mélange présent après la neutralisation de l'étape f).

3. Procédé selon la revendication 2, dans lequel dans l'étape a) on utilise en tant que sel de métal alcalin ou de métal alcalino-terreux de l'acide ascorbique le sel de sodium ou de potassium, ou, respectivement, le sel de calcium, et en tant qu'ester alkylique inférieur ou sel de métal alcalin de l'acide gras l'ester méthylique ou éthylique, ou, respectivement, le sel de sodium.

4. Procédé selon l'une quelconque des revendications 1 à 3, dans lequel l'acide gras est un acide alcanoïque en C₄-C₂₀ saturé, de préférence l'acide palmitique, ou un acide gras en C₄-C₂₀ insaturé comprenant une à trois doubles liaisons éthyléniques, de préférence un acide gras polyinsaturé.

5. Procédé selon l'une quelconque des revendications 2 à 4, dans lequel dans l'étape a) l'acide gras ou son ester alkylique inférieur ou un sel de métal alcalin ou de métal alcalino-terreux est mis à réagir en excès, de préférence en excès d'environ 20% à environ 100% molaire, plus préférablement en excès d'environ 25% molaire, par rapport à la quantité molaire d'acide ascorbique ou de son sel qui est utilisé.

6. Procédé selon l'une quelconque des revendications 2 à 5, dans lequel dans l'étape a) l'acide sulfurique utilisé est l'acide sulfurique à 95% ou une variante de concentration supérieure allant jusqu'à de l'acide sulfurique à 100,5%.

7. Procédé selon l'une quelconque des revendications 1 à 6, dans lequel avant l'extraction avec la diéthylcétone [étape b)] une quantité d'environ trois à six fois en poids d'eau est ajoutée au mélange avant l'extraction avec la diéthylcétone.

8. Procédé selon l'une quelconque des revendications 1 à 7, dans lequel avant l'extraction avec la diéthylcétone [étape b)] de l'eau froide est ajoutée tout en maintenant la température du mélange qui est dilué dans une gamme allant d'environ 0°C à environ 5°C.

9. Procédé selon l'une quelconque des revendications 1 à 8, dans lequel avant l'extraction avec la diéthylcétone [étape b)] de l'eau froide est ajoutée et le mélange dilué moins visqueux est ensuite extrait avec une quantité d'environ trois à environ cinq fois en volume de la diéthylcétone.

10. Procédé selon l'une quelconque des revendications 1 à 9, dans lequel lors de l'hydrolyse des sous-produits d'ester sulfate dans l'extrait de diéthylcétone [étape c)] l'extrait de diéthylcétone séparé est maintenu à une température d'environ 55°C à environ 70°C.

11. Procédé selon l'une quelconque des revendications 1 à 10, dans lequel après le lavage à l'eau et/ou le traitement avec une base faible solide sensiblement insoluble de la phase de diéthylcétone [étape d)] toutes les phases aqueuses issues du lavage et/ou tous les composants solides restant du traitement avec la base sont éliminés.

12. Procédé selon la revendication 11, dans lequel la base est le carbonate solide de calcium ou de magnésium ou XE 654 (Rohm et Haas).

13. Procédé selon l'une quelconque des revendications 2 à 12, dans lequel dans l'étape e) le solvant diéthylcétone est éliminé par évaporation sous pression réduite et à température élevée.

14. Procédé selon l'une quelconque des revendications 1 à 13, dans lequel lors de l'isomérisation des sous-produits d'ester d'acide gras d'ascorbyle-5 en ester d'acide gras d'ascorbyle-6 désiré par catalyse acide dans un solvant organique aprotique non polaire [étape f)] le solvant organique aprotique non polaire est un alcane inférieur, de préférence l'hexane, ou un hydrocarbure aromatique, de préférence le benzène ou le toluène.

15. Procédé selon l'une quelconque des revendications 1 à 14, dans lequel pour l'isomérisation des sous-produits d'ester d'acide gras d'ascorbyle-5 en ester d'acide gras d'ascorbyle-6 désiré par catalyse acide dans un solvant organique aprotique non polaire [étape f)] un acide fort supplémentaire est ajouté et celui-ci est un acide minéral, de préférence de l'acide sulfurique (supplémentaire), de l'acide chlorhydrique ou un sulfate d'hydrogène, ou une résine échangeuse d'ions fortement acide.

16. Procédé selon l'une quelconque des revendications 2 à 15, dans lequel dans l'étape g) la neutralisation est effectuée en ajoutant un carbonate de métal alcalinoterreux, de préférence le carbonate de calcium.

17. Procédé selon l'une quelconque des revendications 2 à 16, dans lequel dans l'étape h) l'isolation de l'ester d'acide gras d'ascorbyle-6 à partir du mélange présent après la neutralisation est effectuée en ajoutant de la diéthylcétone à la suspension neutralisée en une quantité pour dissoudre l'ester d'acide gras d'ascorbyle-6, éventuellement en ajoutant également du charbon, en filtrant le mélange chauffé, en refroidissant le filtrat et en isolant le précipité résultant d'ester d'acide gras d'ascorbyle-6, de préférence par filtration.

18. Procédé selon l'une quelconque des revendications 2 à 17, dans lequel le palmitate de L-ascorbyle est fabriqué et isolé par précipitation, et le sous-produit, l'acide palmitique est récupéré à partir de la liqueur mère de la précipitation du palmitate de L-ascorbyle en concentrant ladite liqueur mère.
